# EUROPEAN PATENT APPLICATION

(11) **EP 0 628 634 A2**
(43) Date of publication of application: **14.12.1994**
(21) Application number: 94303653.3
(22) Date of filing: 23.05.1994
(51) Int. Cl.: C12N 15/54, C12N 9/10, C12N 15/73, C12N 1/21

(54) **Process for preparing serine hydroxymethyltransferase**

(30) Priority: 25.05.1993 US 67631
(71) Applicant: ELI LILLY AND COMPANY, Indianapolis, Indiana 46285 (US)
(72) Inventor: Queener, Stephen Wyatt, Indianapolis, Indiana 46208 (US); Zock, Joseph Martin, Greenwood, Indiana 46143 (US)
(74) Representative: Hudson, Christopher Mark

(57) **Abstract**

This invention provides a process for preparing an ultrahigh amount of *Escherichia coli* SHMT in soluble and active form within an *E. coli* host cell, and recombinant DNA vectors and host cells useful therein.

## Description

This invention relates to the field of recombinant DNA technology. The invention provides a process for the production of serine hydroxymethyltransferase (SHMT).

Serine hydroxymethyltransferase (SHMT) is known to catalyze several chemical reactions of amino acids in microorganisms, plants and animals. SHMT enzymatic reactions include cleavage, condensation, transamination, and racemization reactions (Schirch, L., 1982, Adv. Enzymol. Relat. Areas Mol. Biol. 53:83 and Schirch et al., 1968, J. Biol. Chem. 243:5651.) The natural biological roles of SHMT involve the transfer of one-carbon units to glycine or from serine and the retroaldol cleavage of 1-amino-2-hydroxy acids such as threonine and allo-threonine to generate an aldehyde and glycine.

One of the sources of SHMT is *Escherichia* coli. The SHMT enzyme is the product of the E. coli *gly*A gene. Stauffer et al., 1981, Gene 14:63-72, describe the cloning of the *gly*A gene which encodes SHMT in *Escherichia coli*. The nucleotide sequence for the *gly*A gene has been determined and the amino acid sequence for the SHMT enzyme has been proposed by Plamann et al., 1983, Nucleic Acid Research 11:2065-2075. The *gly*A gene has been overexpressed in *E. coli* host cells at a level 17-26 fold over the wild type and the overproduced enzyme has been isolated and purified (Shirch et al., 1985, J. Bacteriol. 163(1):1-7). However, this level of SHMT production is not commercially practical for large scale enzymatic processes which employ high concentrations of SHMT.

When producing SHMT at high levels, it would be advantageous to allow production of the SHMT enzyme in soluble and active form, attributes that would provide for ease in the purification of the protein. However, many proteins that are expressed at high levels in *Escherichia coli* are often produced in granular form. These proteins are inactive and must be taken through a costly and time consuming process which solubilizes and refolds the protein in proper conformation prior to use. The present invention provides a recombinant DNA process for preparing ultrahigh levels of *Escherichia coli* SHMT in *Escherichia coli* cells in soluble and active form.

The present invention provides a process for preparing an ultrahigh amount of *Escherichia coli* SHMT in soluble and active form within an *E. coli* host cell, said method comprising:
a) Transforming the *E. coli* host cell with a recombinant DNA expression vector comprising:
   i) a transcriptional activating sequence;
   ii) a translational activating sequence; and
   iii) a DNA sequence encoding *E. coli* SHMT;
   such that i) ii) and iii) are operably linked to provide expression of *E. coli* SHMT; and
b) culturing the transformed *E. coli* host cell under conditions that allow for the expression of an ultrahigh amount of *E. coli* SHMT.

The invention further provides recombinant DNA vectors and transformed host cells that are useful in the above process.

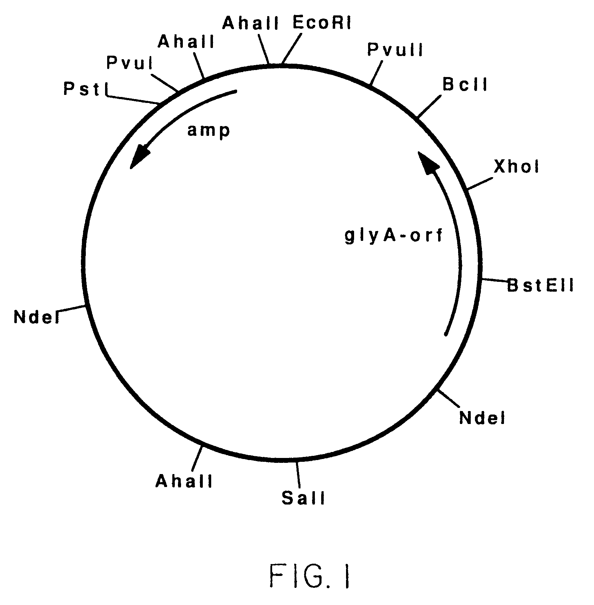

Figure 1 is a restriction enzyme site and function map of plasmid pGS29.

Figure 2 is a restriction enzyme site and function map of plasmid pHKY390.

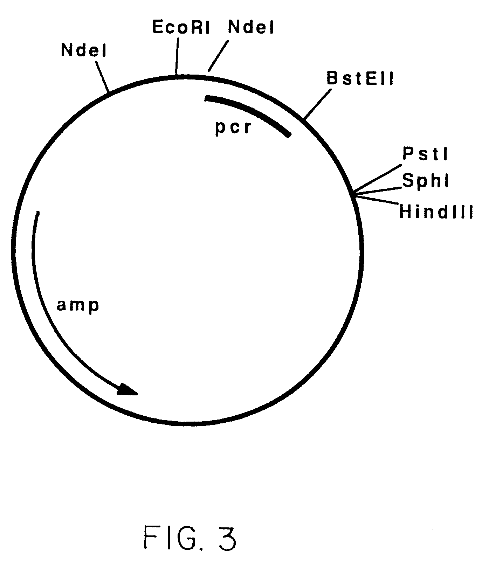

Figure 3 is a restriction enzyme site and function map of plasmid pV-pcrB.

Figure 4 is a restriction enzyme site and function map of plasmid pZPl-glyA.

The restriction enzyme site and function maps presented in the drawings are approximate representations of the recombinant DNA vectors discussed herein. The restriction site information is not exhaustive, there may be more restriction enzyme sites of a given type than are actually shown on the map.

amp - ampicillin resistance phenotype or gene conferring the same.

cI857 - a gene encoding a temperature sensitive repressor of the bacteriophage lambda pL promoter.

Coding sequence - the sequence of DNA in a gene that encodes the amino acid residue sequence of the protein expressed from the gene.

Gene - a segment of DNA that comprises a promoter, translational activating sequence, coding sequence, and 3' regulatory sequences, positioned to drive expression of the gene product.

kan - kanamycin resistance phenotype or gene conferring the same.

orf - open reading frame.

pL - leftward promoter from bacteriophage lambda.

Promoter - a DNA sequence that directs or initiates the transcription of DNA.

Recombinant DNA Expression Vector - any autonomously replicating or integrating agent, including but not limited to plasmids, comprising a promoter and other regulatory sequences positioned to drive expression of a DNA segment that encodes a polypeptide or RNA.

SHMT - serine hydroxymethyltransferase.

Tet - tetracycline resistance phenotype or gene conferring the same.

Transformant - a recipient host cell that has undergone transformation.

Transformation - the introduction of DNA into a recipient host cell that changes the genotype of the recipient cell.

Transcriptional activating sequence - a promoter.

Translational activating sequence - a regulatory DNA sequence that, when transcribed into mRNA, promotes translation of mRNA into protein.

Ultrahigh amount of *Escherichia coli* SHMT - recombinant host cells which produce greater that 40% of total soluble protein as active SHMT (SHMT activity being determined by the 3' phenylserine to benzaldehyde assay described in Example 5).

The present invention provides a process for preparing an ultrahigh amount of *Escherichia coli* SHMT in soluble and active form within an *E. coli* host cell. The process allows ultrahigh expression of the SHMT such that up to 40 to 50% of total water soluble protein of the transformed host cell is SHMT in soluble and active form. This level of gene expression for the production of active SHMT is 7.5-fold higher than levels previously described (Shirch et al., 1985, J. Bacteriol. 163(1):1-7).

A recombinant DNA expression vector that will increase the intracellular concentration of *Escherichia coli* SHMT requires the following elements: 1) *E. coli* SHMT encoding DNA; and 2) a promoter and translational activating sequence that are functional in the *E. coli*, such that these elements are positioned in the correct orientation and position to drive expression of the *E. coli* SHMT DNA. An *E. coli* SHMT expression vector could also comprise an antibiotic resistance-conferring gene or some other element that provides a means of selecting for host cells which contain the vector, but such selectable elements may neither be necessary nor desired when the vector integrates into the chromosomal DNA of the host cell.

The DNA sequence of the *Escherichia coli gly*A gene which encodes *E. coli* SHMT is provided by Plamann et al., 1983, Nucleic Acids Research 11(7):2065. Plamann et al. also describe the plasmid pGS29 which contains the *E.coli gly*A gene. As described below, the plasmid pGS29 was the source of the DNA encoding the *E. coli gly*A gene in the construction of the vectors of the present invention.

An illustrative plasmid that is useful in the present invention is plasmid pZPl-glyA, which was constructed as follows. The NdeI-BamHI restriction enzyme fragment of plasmid pHKY390 (described below), which contains the kanamycin resistance gene, was replaced with a NdeI-BclI restriction enzyme fragment containing the *gly*A open reading frame (ORF) from plasmid pGS29 in a two step process. An NdeI restriction enzyme site was introduced at the ATG start (translational activating sequence) of the *gly*A gene in plasmid pGS29 by the Polymerase Chain Reaction (PCR) as described in Example 2. The *Escherichia coli gly*A open reading frame DNA was then cloned into plasmid pHKY390 on an NdeI-BclI restriction enzyme fragment from plasmid pGS29 to form plasmid pX4, as described in Example 3. The NdeI-BclI restriction enzyme fragment from plasmid pGS29 contained the entire ORF and the native *gly*A promoter sequence.

The native *gly*A promoter DNA sequence was then deleted from plasmid pX4 on a NdeI-BstEII restriction enzyme fragment. This fragment was replaced by a PCR-modified NdeI-BstEII from plasmid pV-pcrB, the construction of which is described in Example 2. The resulting plasmid was pZPl-glyA. Plasmid pZPl-glyA contains the *gly*A ORF in correct alignment to utilize a modified bacteriophage lambda pL promoter of plasmid pHKY390 to drive transcription of the SHMT-encoding DNA.

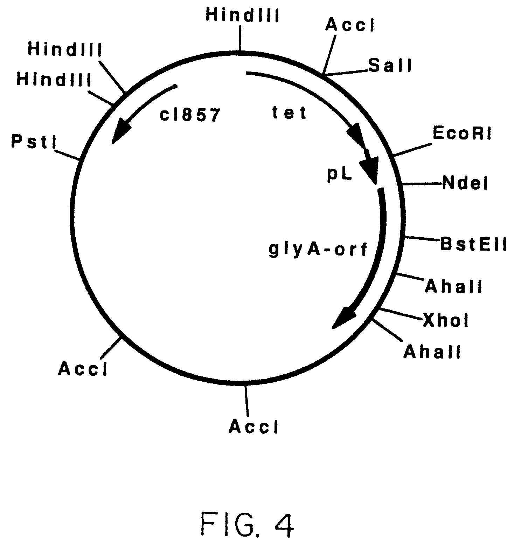

Plasmid pHKY390 is a 6.87 kb plasmid derived from plasmid pBR322. A restriction enzyme and function map of plasmid pHKY390 is shown in Fig. 4. The plasmid contains a region of DNA derived from plasmid pBR322 which allows replication and maintenance of the plasmid in *Escherichia coli*. In this region of plasmid pBR332 DNA, the ampicillin resistance gene was replaced by DNA encoding the bacteriophage lambda cI857 repressor. The lambda cI857 repressor is a temperature sensitive repressor of the bacteriophage lambda promoter. At low temperatures (30°C-35°C) transcription from the modified bacteriophage lambda pL promoter is repressed because the cI857 repressor protein binds to the operator regions of the promoter and blocks transcription. At higher temperatures (36°C-42°C) the repressor protein is disrupted and cannot block transcription from the promoter.

The modified bacteriophage lambda pL promoter in pHKY390 is situated downstream of the tetracycline resistance gene. This promoter is immediately adjacent to the ORF from a kanamycin resistance gene and the promoter controls transcription of that ORF via a two cistron assembly (Schoner et al., 1990, Methods in Enzymology: Gene Expression Technology 185:94-1-3). Plasmid pHKY390 was constructed so that the open reading frame of the kanamycin resistance gene could easily be removed by digestion with the restriction enzymes NdeI and BamHI. Any ORF with a NdeI restriction site at its ATG translation start codon and a BamHI compatible restriction site at or downstream of its translation termination codon could be cloned into plasmid pHKY390. The resulting inserted ORF would be aligned correctly for expression from the modified bacteriophage lambda pL promoter. *Escherichia coli* RV308/pHKY390 was deposited in the permanent culture collection of the Northern Regional Research Laboratory (NRRL), United States Department of Agriculture Service, Peoria, IL 61604, on January 31, 1992, and is available under accession number B-18946. Plasmid pHKY390 can be isolated from *E. coli* RV308/pHKY390 by plasmid isolation techniques that are well known in the art.

As an alternative to the above cloning scheme, the DNA sequence of *Escherichia coli gly*A may be prepared for use in the vectors of the present invention by a variety of other methods, including DNA synthesis, cDNA cloning, genomic cloning, polymerase chain reaction (PCR) technology, or a combination of these approaches. These and other techniques are described by Maniatis, et al. Molecular Cloning: A Laboratory Manual, Cold Spring Harbor Press, Cold Spring Harbor Laboratory, Cold Spring Harbor, New York (1989), or Current Protocols in Molecular Biology (F. M. Ausbel et al., 1989). The contents of both of these references are incorporated herein by reference.

The DNA sequences of *Escherichia coli gly*A open reading frame can be synthesized using commercially available methods and equipment. For example, the solid phase phosphotriester method can be used to produce the DNA sequences of this invention. The DNA sequences can be synthesized by the modified phosphotriester method using fully protected DNA building blocks. Such synthetic methods can be carried out in substantial accordance with the procedure of Itakura, et al., 1977, Science 198:1056 and Crea, et al., Proc. Natl. Acad. Sci. U.S.A. 75:575, and Narang, et al., 1980, Methods in Enzymology 68:90. In addition to manual procedures, the DNA sequences can be synthesized using automated synthesizers such as the ABS 380A DNA Synthesizer (Applied Biosystems, 850 Lincoln Centre Drive, Foster City, CA 94404). The DNA sequence can also be generated by the polymerase chain reaction. See, for example, United States Patent Nos. 4,800,159 and 4,683,202, and European Patent Publication No. 0258017, published March 2, 1987.

For example, the following DNA sequence which encodes the *Escherichia coli gly*A open reading frame and has NdeI and BclI compatible ends can be synthesized and cloned into the large NdeI-BamHI DNA fragment of plasmid pHKY390 to create plasmid pZPl-glyA. The 5' to 3' strand of the following double stranded DNA fragment is designated SEQ. ID. NO 1. The 3' to 5' strand of the following double stranded DNA fragment is designated SEQ. ID. NO 2.

5'-T ATG TTA AAG CGT GAA ATG AAC ATT GCC GAT TAT GAT
3'-   AC AAT TTC GCA CTT TAC TTG TAA CGG CTA ATA CTA

GCC GAA GTC TGG CAG GCT ATG GAG CAG GAA AAA GTA CGT CAG
CGG CTT CAG ACC GTC CGA TAC CTC GTC CTT TTT CAT GCA GTC

GAA GAG CAC ATC GAA CTG ATC GCC TCC GAA AAC TAC ACC AGC
CTT CTC GTG TAG CTT GAC TAG CGG AGG CTT TTG ATG TGG TCG

CCC CGC GTA ATG CAG GCG CAG GGT TCT CAG CTG ACC AAC AAA
GGG GCG CAT TAC GTC CGC GTC CCA AGA GTC GAC TGG TTG TTT

TAT GCT GAA GGT TAT CCG GGC AAA CGC TAC TAC GGC GGT TGC
ATA CGA CTT CCA ATA GGC CCG TTT GCG ATG ATG CCG CCA ACG

GAG TAT GTT GAT ATC GTT GAA CAA CTG GCG ATC GAT CGT GCG
CTC ATA CAA CTA TAG CAA CTT GTT GAC CGC TAG CTA GCA CGC

AAA GAA CTG TTC GGC GCT GAC TAC GCT AAC GTC CAG CCG CAC
TTT CTT GAC AAG CCG CGA CTG ATG CGA TTG CAG GTC GGC GTG

TCC GGC TCC CAG GCT AAC TTT GCG GTC TAC ACC GCG CTG CTG
AGG CCG AGG GTC CGA TTG AAA CGC CAG ATG TGG CGC GAC GAC

GAA CCA GGT GAT ACC GTT CTG GGT ATG AAC CTG GCG CAT GGC
CTT GGT CCA CTA TGG CAA GAC CCA TAC TTG GAC CGC GTA CCG

GGT CAC CTG ACT CAC GGT TCT CCG GTT AAC TTC TCC GGT AAA
CCT GTG GAC TGA GTG CCA AGA GGC CAA TTG AAG AGG CCA TTT

CTG TAC AAC ATC GTT CCT TAC GGT ATC GAT GCT ACC GGT CAT
GAC ATG TTG TAG CAA GGA ATG CCA TAG CTA CGA TGG CCA GTA

ATC GAC TAC GCC GAT CTG GAA AAA CAA GCC AAA GAA CAC AAG
TAG CTG ATG CGG CTA GAC CTT TTT GTT CGG TTT CTT GTG TTC

CCG AAA ATG ATT ATC GGT GGT TTC TCT GCA TAT TCC GGC GTG
GGC TTT TAC TAA TAG CCA CCA AAG AGA CGT ATA AGG CCG CAC

GTG GAC TGG GCG AAA ATG CGT GAA ATC GCT GAC AGC ATC GGT
CAC CTG ACC CGC TTT TAC GCA CTT TAG CGA CTG TCG TAG CCA

GCT TAC CTG TTC GTT GAT ATG GCG CAC GTT GCG GGC CTG GTT
CGA ATG GAC AAG CAA CTA TAC CGC GTG CAA CGC CCG GAC CAA

GCT GCT GGC GTC TAC CCG AAC CCG GTT CCT CAT GCT CAC GTT
CGA CGA CCG CAG ATG GGC TTG GGC CAA GGA GTA CGA GTG CAA

GTT ACT ACC ACC ACT CAC AAA ACC CTG GCG GGT CCG CGC GGC
CAA TGA TGG TGG TGA GTG TTT TGG GAC CGC CCA GGC GCG CCG

GGC CTG ATC CTG GCG AAA GGT GGT AGC GAA GAG CTG TAC AAA
CCG GAC TAG GAC CGC TTT CCA CCA TCG CTT CTC GAC ATG TTT

AAA CTG AAC TCT GCC GTT TTC CCT GGT GGT CAG GGC GGT CCG
TTT GAC TTG AGA CGG CAA AAG GGA CCA CCA GTC CCG CCA GGC

TTG ATG CAC GTA ATC GCC GGT AAA GCG GTT GCT CTG AAA GAA
AAC TAC GTG CAT TAG CGG CCA TTT CGC CAA CGA GAC TTT CTT

GCG ATG GAG CCT GAG TTC AAA ACT TAC CAG CAG CAG GTC GCT
CGC TAC CTC GGA CTC AAG TTT TGA ATG GTC GTC GTC CAG CGA

AAA AAC GCT AAA GCG ATG GTA GAA GTG TTC CTC GAG CGC GGC
TTT TTG CGA TTT CGC TAC CAT CTT CAC AAG GAG CTC GCG CCG

TAC AAA GTG GTT TCC GGC GGC ACT GAT AAC CAC CTG TTC CTG
ATG TTT CAC CAA AGG CCG CCG TGA CTA TTG GTG GAC AAG GAC

GTT GAT CTG GTT GAT AAA AAC CTG ACC GTT AAA GAA GCA GAC
CAA CTA GAC CAA CTA TTT TTG GAC TGG CAA TTT CTT CGT CTG

GCC GCT CTG GGC CGT GCT AAC ATC ACC GTC AAC AAA AAC AGC
CGG CGA GAC CCG GCA CGA TTG TAG TGG CAG TTG TTT TTG TCG

GTA CCG AAC GAT CCG AAG AGC CCG TTT GTG ACC TCC GGT ATT
CGT GGC TTG CTA GGC TTC TCG GGC AAA CAC TGG AGG CCA TAA

CGT GTA GGT ACT CCG GCG ATT ACC CGT CGC GGC TTT AAA GAA
GCA CAT CCA TGA GGC CGC TAA TGG GCA GCG CCG AAA TTT CTT

GCC GAA GCG AAA GAA CTG GCT GGC TGG ATG TGT GAC GTG CTG
CGG CTT CGC TTT CTT GAC CGA CCG ACC TAC ACA CTG CTC GAC

GAC AGC ATC AAT GAT GAA GCC GTT ATC GAG CGC ATC AAA GGT
CTG TCG TAG TTA CTA CTT CGG CAA TAG CTC GCG TAG TTT CCA

AAA GTT CTC GAC ATC TGC GCA CGT TAC CCG GTT TAC GCA
TTT CAA GAG CTG TAG ACG CGT GCA ATG GGC CAA ATG CGT

TAAGCGAAAC GGTGATTTGC TGTCAATGTG CTCGTTGTTC ATGCCGGATG
ATTCGCTTTG CCACTAAACG ACAGTTACAC GAGCAACAAG TACGGCCTAC

CGGCGTGAAC GCCTTATCCG GCCTACAAAA CTTTGCAAAT TCAATATATT
GCCGCACTTA CGGAATAGGC CGGATGTTTT GAAACGTTTA AGTTATATAA

GCAATCTCCG TGTAGGCCTG ATAAGCGTAG CGCATCAGGC AATTTTTCGT
CGTTAGAGGC ACATCCGGAC TATTCGCATC GCGTAGTCCG TTAAAAAGCA

TTAT -3'
AATACTAG-5'

The process of the present invention is not limited to the particular vectors exemplified herein. Instead, the present invention comprises a process wherein a variety of expression vectors can be used to express *Escherichia coli* SHMT in an *E. coli* host cell to ultrahigh levels. Expression in prokaryotic cells is described by Maniatis et al. (1989), and Kaufmann, Genetic Engineering Principles and Methods, ed. J. K. Setlow, Plenum Press 9:155, (1988).

The present invention is not limited to a particular transcriptional activating sequences and translational activating sequence to drive expression of *Escherichia coli* SHMT. The invention comprises the use of transcriptional activating sequences and translational activating sequences that function in *E. coli* and that can be used to express *E. coli* SHMT to ultrahigh levels in *E. coli*. Many promoter and translational activating sequences functional in *E. coli* are known and are suitable for driving expression of *E. coli* SHMT in *E. coli*. Such transcriptional and translational activating sequences include, but are not limited to, the *lpp*, *lac*, *trp*, *tac*, lpL, and lpR promoter and translational activating sequences. In addition, modified bacteriophage lambda promoters such as those disclosed in U.S. Patent Application Serial No. 07/739,280, which is incorporated herein by reference, are useful in the present invention.

The following examples are intended to assist in the further understanding of the invention. Particular materials employed, species, and conditions are intended to be further illustrative of the invention and not limiting the reasonable scope thereof. Procedures for the manipulation and analysis of DNA were performed essentially as described by Sambrook et al., 1989, Molecular Cloning: a Laboratory Manual, Cold Spring Harbor Laboratory Press, Cold Spring Harbor, NY. Conditions for restriction enzyme reactions were those recommended by the manufacturers (Boehringer Mannheim (BM), Indianapolis, IN; New England Biolabs (NEB), Beverly, MA; Bethesda Research Labs (BRL), Gaithersburg, MD.

### EXAMPLE 1

### Preparation of Escherichia coli Host Cells Transformed with Plasmid pGS29

The nucleotide sequence of the *Escherichia coli* glyA gene is known (Plamann et al., 1983, Nucleic Acid Research 11:2065-2075. However, as a matter of convenience, plasmid pGS29 isolated from *Escherichia coli* GS245/pGS29 was employed as a starting material for the vectors of this invention. *Escherichia coli* GS245/pGS29 was described by Plamann and Stauffer, 1983, Gene 22:9-18.

A portion of an *Escherichia coli* GS245/pGS29 colony grown on L-agar containing 50 µg/ml ampicillin was transferred to one liter of L-broth containing 50 µg/ml ampicillin and incubated in an air-shaker at 37°C for about 16 hours. Cells were harvested in 500 ml bottles in a Beckman JA-10 rotor (Beckman Insts. Inc., Fullerton, CA. 92634 (Beckman)) at 8000 rpm for 10 minutes at 4°C. The cell pellet was washed with TE-8.0 (10 mM Tris-HCl (pH 8.0) 1 mM ethylenediaminetetraacetic acid (EDTA)), collected, and resuspended in 50 mM Tris-HCl (pH 8.0) and 25% sucrose to a total volume of 10 ml. One milliliter of 20 mg/ml lysozyme (Sigma Chemical Co., St. Louis, MO 63178) in 25 mM Tris-HCl, pH 8.0, was added with stirring and the mixture was then incubated on ice for 30 minutes. Four milliliters of 200 mM EDTA was added with subsequent incubation on ice for 10 minutes followed by the addition of 15 ml of Brij/DOC lysing solution (1% Brij 58; 0.4% deoxycholate; 50 mM Tris-HCl, pH 8.0; 60 mM EDTA). Tubes were gently inverted to mix and incubated on ice for 15-30 minutes. Cell debris was removed by centrifugation for 1 hour at 18,000 rpm in a Beckman JA-20 rotor. Supernatant was decanted yielding approximately 30 ml to which was added 150 µl of 10 mg/ml RNAse A (Sigma). After a 1 hour incubation at 37°C, 150 µl of 10 mg/ml Proteinase K (Boehringer Mannheim) was added, followed by another 1 hour incubation at 37°C. DNA was precipitated by the addition of 1/10 volume of 3 M sodium acetate, pH 7.0, followed by 3X volumes of ice cold absolute ethanol. DNA was recovered by centrifugation in a Beckman JA-14 rotor at 8,000 rpm for 30 minutes. The air dried pellet was resuspended in TE-8.0 to a total volume of 9 ml, to which was added 9 g of cesium chloride (Boehringer Mannheim) and 0.5 ml of 10 mg/ml ethidium bromide. The resulting solution was loaded into two 5.1 ml Quik-seal tubes (Beckman) and centrifuged at 65,000 rpm in a Beckman VTi65.2 ultracentrifuge rotor for 6 hours. The plasmid band was visualized under ultraviolet light and was removed by syringe. The resulting DNA solution was extracted with salt-saturated isopropanol to remove the ethidium bromide and dialyzed 16 hours against 1000x volumes TE-8.0. DNA solutions were stored at -20°C until needed.

Transformation competent *Escherichia coli* DH5α cells were obtainbed from Gibco BRL (Gaithersburg, MD) and were transformed with plasmid pGS29 in accordance with the manufacturer's protocol. Transformants were selected on L-agar containing 50 µg/ml ampicillin. Transformants containing plasmid pGS29 were verified by plasmid sizing on horizontal gel electrophoresis. A representative *E. coli* DH5α transformant bearing plasmid pGS29 was designated D.1.

Alternatively, plasmid pGS29 was used to transform *Escherichia coli* RV308. A culture of *Escherichia coli* RV308 was deposited in the permanent culture collection of the Northern Regional Research Laboratory (NRRL), United States Department of Agriculture Service, Peoria, IL 61604, on September 28, 1983, and is available under accession number B-15624.

*Escherichia coli* RV308 cells were prepared for transformation as follows. A sample of *E. coli* RV308 cells were grown in L-broth (10 g tryptone, 10 g NaCl, and 5 g yeast extract per liter) to an O.D.₅₉₀ of about 0.5 absorbance units, and the cells were collected by centrifugation. The cell pellet was resuspended in 25 ml of cold 100 mM CaCl₂ and incubated on ice for 25 minutes. The cells were once again collected by centrifugation, and the pellet was resuspended in 2.5 ml of cold 100 mM CaCl₂ and incubated overnight. Competent cells were transformed or stored directly at -70°C until ready for use in transformations.

To transform competent *Escherichia coli* RV308 cells with plasmid pGS29, one hundred microliters of the competent cell suspension in a polypropylene tube were removed from storage at -70°C and allowed to thaw on ice. The cells were gently mixed and five microliters (µl) of a solution of plasmid pGS29 (1 ng/µl) was added and the resulting solution incubated on ice for 30 minutes. The tube was transferred to a 42°C water bath and incubated without shaking for 45 seconds. After the heat-shock treatment, the tube was placed on ice for 2 minutes. The tube was removed from ice and 0.9 ml of S.O.C. medium (2% Bactotryptone; 0.5% yeast extract; 10 mM NaCl; 2.5 mM KCl; 10 mM MgCl₂; 10 mM MgSO₄; 20 mM glucose; in distilled water) at room temperature was added. The solution was incubated for 1 hour at 37°C while shaking at 225 revolutions per minute (rpm). Aliquots of the cell mixture were plated on L-agar (L-broth with 15 grams/liter (g/L) agar) plates containing 50 µg/ml ampicillin, and the plates were incubated at 37°C. Transformants were verified by selection for ampicillin resistance and by plasmid sizing on horizontal gel electrophoresis. Plasmid DNA from the clones was isolated and confirmed by restriction enzyme analysis. Representative transformants of *Escherichia coli* RV308 bearing plasmid pGS29 were designated R.1.

### EXAMPLE 2

### Construction of Plasmid pV-pcrB

### A. Construction of a PCR-Modified/Amplified Fragment

A PCR-modified/amplified fragment that contained the 5' end of the *Escherichia coli gly*A gene altered to have an NdeI site at the ATG start codon and *gly*A encoding DNA inclusive of the BstEII site of the *gly*A gene was constructed.

The DNA oligonucleotide primers used in the amplification were as follows. The forward primer was a 26 base oligonucleotide with the sequence: 5'-GATGCATATGTTAAAGCGTGAAATGA-3' (SEQ. ID. NO: 3).
SEQ. ID. NO: 3 was designed to hybridize across the ATG transcriptional start codon of the *Escherichia coli gly*A gene. The bold type represents the NdeI recognition sequence and the underlined bases were those changed from the original *E. coli gly*A gene sequence.

The reverse oligonucleotide primer was a 15 base pair DNA oligonucleotide with the sequence: 5'-CCGGAGAACCGTGAG-3' (SEQ. ID. NO:4).
SEQ. ID. NO:4 was designed to hybridize immediately downstream of the single BstEII site in the *Escherichia coli gly*A gene sequence. Both oligonucleotides were synthesized on an ABI 380B DNA synthesizer and purified with an Oligonucleotide Purification Cartridge®, (Applied Biosystems Inc., Foster City, CA). Materials necessary for carrying out the PCR reaction with these primers were obtained in a GeneAmp™ DNA Amplification Reagent Kit (Perkin Elmer Cetus, Norwalk, CT).

The reaction conditions were as follows: One nanogram of template DNA (plasmid pGS29); 1.0 mM SEQ. ID. NO:3; 1.0 mM SEQ. ID. NO:4; 200 mM each dATP (deoxyadenosine 5'-triphosphate), dCTP (deoxycytosine 5'-triphosphate), dGTP (deoxyguanosine 5'-triphosphate), and TTP (thymidyl 5'-triphosphate); 1*X* reaction buffer (10 mM Tris-HCl (pH 8.3); 50 mM KCl; 1.5 mM MgCl₂; 0.01%[w/v] gelatin); and 2.5 units Taq Polymerase in a 100 µl final volume. The reaction mixture (in a 0.5 ml polypropylene tube) was overlaid with 100 µl mineral oil to prevent evaporation was placed in a DNA Thermal Cycler (Perkin Elmer Cetus). Thermocycler settings were as follows: one cycle of 2 minutes at 94°C; thirty cycles of a) 1 minute at 94°C b) 2 minutes at 50°C, and c) 3 minutes at 72°C; one cycle of 7 minutes at 72°C; and a final (storage) cycle at 4°C.

The 403 base pair PCR-modified/amplified DNA fragment was converted to a 380 base pair BstEII-NdeI DNA fragment by digestion with 50 units of BstEII enzyme in a reaction containing 1X BamHI/BstEII buffer (10 mM Tris-HCl (pH 8.0); 5 mM magnesium chloride; 100 mM NaCl; and 1 mM 2-mercaptoethanol) for 2 hours at 60°C. The fragment was gel-isolated as follows. The digested DNA was electrophoresed on an 1.5% agarose gel in 1X TAE buffer (40 mM Tris-Acetate; 1 mM EDTA). The gel was stained in a dilute (1 mg/ml) ethidium bromide solution and the DNA bands visualized under long-wave ultraviolet (UV) light. The 380 base pair BstEII digested PCR-modified/amplified DNA fragment was located and excised from the gel with a fresh scalpel blade. Elution of the DNA fragment from the gel slice was performed according to the protocol of Maniatis et al. (T. Maniatis, J. Sambrook, and E. F. Fritsch, 1989, Molecular Cloning: A Laboratory Manual Cold Springs Harbor Laboratory Press, pp. 6.28) with minor adjustments. Briefly, the gel slice was put into a dialysis bag with about 200 µl 1/5X TAE buffer, sealed with clips, and electrophoresed in 1/5X TAE buffer for about 3 hours. The contents of the dialysis bag, including a 400 µl wash with 1/5X TAE were mixed with 2.4 ml low salt buffer (0.2 M NaCl; 20 mM Tris-HCl, pH 7.5; 1.0 mM EDTA) and loaded onto a prepared ELUTIP-d column (Schleicher & Schuell, Keene, NH) following the supplied protocol. After washing with low salt buffer the DNA was eluted from the column with two 400 µl volumes of high salt buffer (1.0 M NaCl; 20 mM Tris-HCl, pH 7.5; 1.0 mM EDTA) and precipitated by addition of 800 µl of ice-cold absolute ethanol followed by centrifugation at 14,000 rpm in an Eppendorf 5415C microfuge for 40 minutes. The two air-dried pellets were combined by dissolving in 20 µl TE, pH 7.5 and the solutions were stored at -20°C until ligation. This fragment contained amino-terminal region of the *Escherichia coli gly*A gene modified by PCR to have an NdeI Site at its ATG translation initiation start site.

### B. Construction of a Sau3AI-HphI Linker DNA Fragment

To isolate a Sau3AI-HphI linker fragment two separate digestions and isolations were performed. First, approximately 20 µg of plasmid pUC19 (available from BRL) was digested with 50 units of HphI restriction enzyme (NEB) in a reaction containing 1X HphI buffer (20 mM Tris-acetate (pH 7.9); 50 mM potassium acetate; 10 mM magnesium acetate; and 1 mM dithiothreitol) for 2 hours at 37°C. The appropriate 416 base pair HphI restriction fragment was gel-isolated as in Example 2A. The fragment was then digested with 50 units of Sau3AI restriction enzyme (BM) in a reaction containing 1X Sau3AI buffer (33 mM Tris-acetate (pH 7.9); 10 mM magnesium acetate; 66 mM potassium acetate; 0.5 mM dithiothreitol) for 2 hours at 37°C. The 234 base pair Sau3AI-HphI DNA fragment was gel-isolated as in Example 2A.

### C. Construction of Plasmid pV-pcrA

Approximately 5 µg of plasmid pUC19 DNA were digested with 50 units of restriction enzyme AccI in a reaction containing 1X AccI buffer (33 mM Tris-acetate (pH 7.9); 10 mM magnesium acetate; 66 mM potassium acetate; and 0.5 mM DTT) for 2 hours at 37°C. Linearized plasmid pUC19 was gel isolated by the method described in Example 2A.

Approximately 10 µg of plasmid pGS29 were digested with 50 units of AhaII enzyme (NEB) in a reaction containing 1X AhaII buffer (10 mM Tris-HCl (pH 7.9); 50 mM NaCl; 10 mM MgCl₂; 1 mM DTT; and 100 mg/ml bovine serum albumin) for 2 hours at 37°C. The 2.0 kb AhaII fragment was isolated by the method described in Example 2A.

Three microliters of the 2.0 kb AhaII fragment from plasmid pGS29 and 1 µl of plasmid pUC19 (2.8 kb) linearized with AccI were added to a 15 µl reaction containing 1X ligase buffer and about 5 units of T4 ligase (BM). The reaction mixture was incubated at 14°C for 16 hours. The reaction products were transformed into *Escherichia coli* DH5α cells as described in Example 1. Plasmids from the transformants were screened for orientation of the AhaII fragment in the plasmid. A plasmid containing the BstEII site proximal to the PstI site and distal to the BamHI site of plasmid pUC19 was chosen as plasmid pV-pcrA. Plasmid from this clone was isolated as in Example 1.

To obtain the desired BstEII-BamHI vector backbone fragment, approximately 10 µg of plasmid pV-pcrA were digested with 50 units BamHI enzyme (BM) in a reaction containing 1X BamHI/BstEII buffer for 2 hours at 37°C. BstEII (50 units) was added to the reaction and the temperature raised to 60°C and held for 2 hours. The BstEII-BamHI vector backbone fragment was gel-isolated in accordance with the method of Example 2A.

### D. Final Construction of Intermediate Plasmid pV-pcrB

Intermediate plasmid pV-pcrB was obtained by ligating the BstEII-digested 380 base pair PCR-modified/amplified DNA fragment prepared in Example 2A with the linker fragment prepared in Example 2B and with the vector backbone fragment prepared in Example 2C.

One microliter (about 50 ng) of the vector backbone fragment, 2 µl (about 100 ng) of the Sau3AI-HphI DNA fragment, and 6 µl (about 100 ng) of the BstEII-digested PCR-modified/amplified DNA fragment were added to a reaction containing 1X ligation buffer with about 5 units of T4 ligase. The mixture was incubated at 14°C for 16 hours and the resulting DNA was transformed into *Escherichia coli* DH5a cells by the method described in Example 1. Transformants were screened by restriction enzyme analysis to select a transformant bearing plasmid pV-pcrB. Plasmid pV-pcrB was extracted and purified from that transformant by the method described in Example 1.

### EXAMPLE 3

### Construction of Plasmid pX4

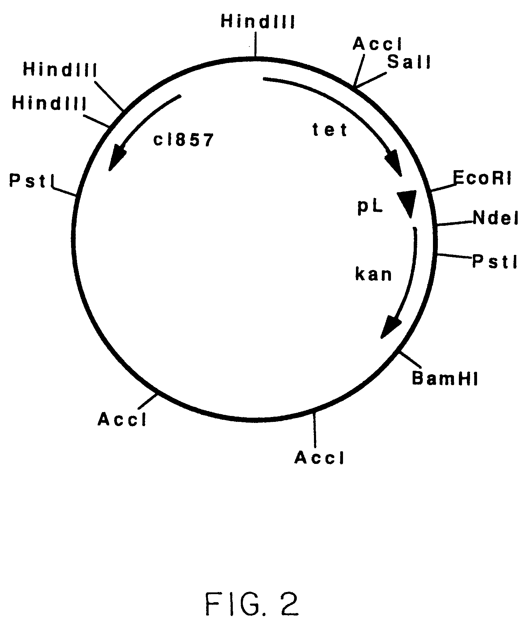

A restriction enzyme site and function map of plasmid pHKY390 is shown in Figure 2. The open reading frame of the kanamycin resistance gene was removed from plasmid pHKY390 as follows. Ten micrograms of plasmid pHKY390 were digested with 50 units of NdeI restriction enzyme in a reaction mixture containing 1X NdeI buffer for 2 hours at 37°C. The digested DNA was precipitated as described above and the resulting DNA pellet was resuspended in 1X BamHI buffer. Fifty units of BamHI restriction enzyme was added, and the mixture was incubated at 37°C for 2 hours. The larger of two NdeI-BamHI DNA fragments of plasmid pHKY390 was gel-isolated according to the procedure described in Example 2A.

The NdeI-BclI DNA fragment, which contained the *Escherichia coli gly*A gene and upstream sequences, was obtained from plasmid pGS29 as follows. Approximately 10 µg of plasmid pGS29 was digested with 50 units NdeI in a reaction containing 1X NdeI buffer for 2 hours at 37°C. The digested DNA was precipitated by the addition of 1/10 volume of 3 M sodium acetate, pH 7.0, and 2.5 volumes of ice-cold absolute ethanol. The DNA was collected by a 30 minute centrifugation. The pellet of DNA was resuspended and dissolved in 1X BclI buffer (10 mM Tris-HCl.(pH 7.5); 10 mM magnesium chloride; 50 mM sodium chloride; and 1 mM dithioerythritol); 50 units of BclI restriction enzyme was added, and the reaction mixture was incubated for 2 hours at 50°C. The 1.73 kb NdeI-BclI DNA fragment was added directly to a ligation mixture containing the gel-isolated NdeI-BamHI DNA fragment from plasmid pHKY390.

The 1.73 kb NdeI-BclI DNA fragment of plasmid pGS29, which contained the *gly*A open reading frame and upstream sequences, was ligated to the large NdeI-BamHI DNA fragment from plasmid pHKY390 as follows. Five microliters (about 50 ng) of the large NdeI-BamHI DNA fragment obtained from plasmid pHKY390 and 5 µl (about 500 ng) of the NdeI-BclI-digested plasmid pGS29 DNA were added to a reaction containing 1X ligase buffer and 5 units of T4 ligase. The resulting mix was incubated at 14°C for 16 hours and the resulting DNA products transformed into *Escherichia coli* DH5a cells as described in Example 1 with the exception that transformation and incubation were carried out at 30°C. Transformants were selected for tetracycline resistance. Plasmids from the resulting tetracycline resistant transformants were screened by restriction enzyme analysis to find transformants that carried a plasmid with the structure of the desired plasmid pX4. Plasmid pX4 DNA was purified from a selected transformant by the method described in Example 1.

### EXAMPLE 4

### Construction of Plasmid pZPl-glyA

To construct plasmid pZPl-glyA the small NdeI-BstEII DNA fragment of plasmid pX4 was replaced with the small NdeI-BstEII DNA fragment from plasmid pV-pcrB. This was accomplished as follows. Plasmid pX4 (10 µg) was digested with NdeI (50 units) in 1X NdeI buffer for 2 hours at 37°C. The DNA was precipitated as in Example 3 and the precipitated DNA was resuspended in 1X BstEII buffer containing 50 units of BstEII. The reaction mixture was incubated at 60°C for 2 hours and the large DNA fragment was gel-isolated according to Example 2A. Approximately 20 µg of pV-pcrB were digested identically to the digestion of plasmid pX4 and the 373 base pair NdeI-BstEII DNA fragment was gel isolated.

The large NdeI-BstEII DNA fragment obtained from plasmid pX4 and the 373 base pair NdeI-BstEII DNA fragment obtained from plasmid pV-pcrB were ligated to form plasmid pZPl-glyA as follows. Two microliters (about 50 ng) of the fragment from vector pX4 and one microliter (about 150 ng) of the 373 base pair fragment from plasmid pV-pcrB were added to a reaction mixture containing 1X ligase buffer and 5 units of T4 ligase. The resulting reaction mixture was incubated at 14°C for 16 hours.

The ligation reaction mixture was prepared for transformation by diluting 5-fold in sterile TE. Using the diluted DNA, competent *Escherichia coli* DH5α cells were transformed essentially as in Example 3. Tetracycline resistant colonies were selected and plasmids from these colonies were sized by horizontal agarose gel electrophoresis. Transformants that contained a plasmid with the size corresponding to that expected for plasmid pZPl-glyA were selected. Plasmids from these transformants were confirmed to have the structure expected for plasmid pZPl-glyA by restriction enzyme analysis. A representative *E. coli* DH5α/pZPl-glyA transformant was chosen and designated Dgly.

Plasmid pZPl-glyA was isolated from *Escherichia coli* DH5α /pZPl-glyA and used to transform *E. coli* RV308 as described in Example 3. Plasmids from these transformants were confirmed to have the structure expected for plasmid pZPl-glyA by restriction enzyme analysis. A representative *E. coli* RV308/pZPl-glyA transformant was chosen and designated Rgly.

### Example 5

### Enzyme Assay

To facilitate the activity analysis of the *Escherichia coli* recombinants described above, a high performance liquid chromatography (HPLC) assay was developed using 3'-phenylserine as a substrate for SHMT. The SHMT activity was determined by monitoring the conversion of 3'-phenylserine to benzaldehyde. The reaction mixture in a total volume of 1 ml contained 20 mM of 3'-phenylserine and a suitable amount of the enzyme in 20 mM N, N-bis[2-hydroxyethyl]-2-aminoethanesulfonic acid buffer (BES), pH 7.25. The reaction mixture was incubated at 30°C with gentle shaking and, after 5 minutes, was stopped by addition of 25 µl of 10% H₃PO₄. Any particulates were removed by centrifugation and a small portion (usually 10 µl) of the supernatant fraction was analyzed by HPLC. Benzaldehyde was quantitated by using an Apex ODS 3µ C18 column (Jones Chromatography, Littleton, CO), isocratic elution of 1 ml/min with water/acetonitrile (75/25, v/v) and detection at 279 nm. One unit of enzyme activity is defined as the amount of the SHMT required to cause formation of one µmole of benzaldehyde per minute under the reaction conditions. The specific activity is defined as units per mg of protein. The protein content was determined by the standard method of Bradford using bovine serum albumin as the standard.

### EXAMPLE 6

### SHMT Expression

Overnight cultures of *Escherichia coli* RV308, *E. coli* DH5α, *E. coli* RV308/pGS29 (R.1), *E. coli* DH5α/pGS29 (D.1), *E coli* RV308/pZPI-glyA (Rgly), and *E. coli* DH5α /pZPI-glyA (Dgly) (media volume 25% of flask volume) were inoculated from frozen stock into Luria Broth containing 5 µg/ml tetracycline. The cultures were incubated at 30°C for approximately 16 hours on an aerated shaker/incubator at 250 rpm. The optical density (O.D.; 600 nm wavelength) of the overnight culture broth was measured using samples diluted with known volumes of water to achieve a linear relationship between O.D. and diluted sample. The overnight cultures were diluted 1 to 25 into fresh Luria Broth and 5 µg/ml tetracycline. The cultures were allowed to grow to an O.D. (600 nm wavelength) equivalent to half the O.D. of the overnight culture; the temperature of the culture was then shifted to 40°C and incubation with shaking was continued in a water bath or aerated shaker/incubator depending on culture size. The cultures were kept at this temperature for the rest of the experiment. Samples for assay were taken at the time of the temperature shift (time zero) and at various times thereafter. Cells in broth samples were harvested by centrifugation. The harvested cells were washed once in 10 mM Tris-HCl, 1 mM EDTA and 0.1 mM pyridoxal-5'-phosphate (breaking buffer). Five grams of each cell pellet were resuspended in 10 ml breaking buffer and sonicated 3 x 30 seconds at 2°C. The sonicates were clarified by centrifugation at 48,000 x g for 1 hour to produce the crude extracts. SHMT activity for each culture was measured by an HPLC enzyme assay as described in Example 5. Total and specific enzyme activities from the shake-flask cultures of *Escherichia coli* RV308, *E. coli* DH5α, R.1, D.1, Rgly, and Dgly are shown in Table 1.

**Table 1**

| Strain | SHMT Activity | Protein | SHMT Sp. Act. |
|---|---|---|---|
| | (Units/ml) | (mg/ml) | (Units/mg) |
| DH5α | 1.48 | 27 | 0.05 |
| D.1 | 50.84 | 36 | 1.41 |
| Dgly | 92.16 | 31 | 2.97 |
| RV308 | 0.97 | 31 | 0.03 |
| R.1 | 24.6 | 30 | 0.82 |
| Rgly | 44.01 | 33 | 1.33 |

### EXAMPLE 7

### Production of Escherichia coli SHMT

A seed lot was prepared by growing *Escherichia coli* RV308/pZPlglyA (Rgly) at 30°C in L broth with 5 µg/ml tetracycline added, until the desired cell density was reached. One ml aliquots were transferred under sterile conditions to screw-capped freezer vials and preserved in the vapor phase of liquid nitrogen. A vial of the seed lot was removed from storage and the contents transferred equally into 2 flasks each containing 50 ml of L broth with 5 µg/ml tetracycline and incubated at 30°C. The contents of these flasks were used to inoculate a 50 liter stirred reactor containing a modified M-9 glucose-salts medium. This seed vessel was controlled at 30°C, pH 7.0, and dissolved O₂ at greater than 50% until off gas analysis indicated that reasonable cell mass had been achieved. A portion of the broth was then transferred into a 150 liter bioreactor. During the cell mass accumulation phase, temperature was maintained at 30°C and pH controlled at pH 7.0. When fermentation parameters reached a predetermined setpoint (determined by the gas transfer characteristics of each individual bioreactor), the temperature of the fermentor was ramped up to 41°C. At this time recombinant protein synthesis began within the bacterial cells. A slurry of complex amino acids, such as hydrolyzed casein, was bulk fed into the reactor just prior to reaching final temperature. For the remainder of the fermentation, glucose and the complex amino acid slurry were fed at a continuous rate into the reactor. The level fed was sufficient to give and maintain pools of individual amino acids throughout the productive phase. Temperature and pH were controlled throughout the productive phase. The cells containing the correctly folded soluble product were harvested when fermentation parameters such as dissolved oxygen and carbon dioxide evolution rate indicated that fermentation objectives, such as dissolved oxygen rising and carbon dioxide evolution falling, had been met. In 150 liter fermentors, Rgly grew better (i.e. had more total enzyme activity) than Dgly.

SEQUENCE LISTING

1) GENERAL INFORMATION:
(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: PROCESS FOR PREPARING SERINE
HYDROXYMETHYLTRANSFERASE

(iii)NUMBER OF SEQUENCES: 4

(iv) CORRESPONDENCE ADDRESS:
(A) ADDRESSEE: C. M. Hudson
(B) STREET: Erl Wood Manor,
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(2) INFORMATION FOR SEQ ID NO: 1:
(i) SEQUENCE CHARACTERISTICS:
(A) LENGTH: 1406 nucleotides
(B) TYPE: nucleic acid
(C) STRANDEDNESS: single
(D) TOPOLOGY: linear
(ii) MOLECULE TYPE: DNA
(xi) SEQUENCE DESCRIPTION: SEQ ID NO: 1

T ATG TTA AAG CGT GAA ATG AAC ATT GCC GAT TAT GAT GCC 40

GAA CTG TGG CAG GCT ATG GAG CAG GAA AAA GTA CGT CAG GAA 82

GAG CAC ATC GAA CTG ATC GCC TCC GAA AAC TAC ACC AGC CCC 124

CGC GTA ATG CAG GCG CAG GGT TCT CAG CTG ACC AAC AAA TAT 166

GCT GAA GGT TAT CCG GGC AAA CGC TAC TAC GGC GGT TGC GAG 208

TAT GTT GAT ATC GTT GAA CAA CTG GCG ATC GAT CGT GCG AAA 250

GAA CTG TTC GGC GCT GAC TAC GCT AAC GTC CAG CCG CAC TCC 292

GGC TCC CAG GCT AAC TTT GCG GTC TAC ACC GCG CTG CTG GAA 334

CCA GGT GAT ACC GTT CTG GGT ATG AAC CTG GCG CAT GGC GGT 376

CAC CTG ACT CAC GGT TCT CCG GTT AAC TTC TCC GGT AAA CTG 418

TAC AAC ATC GTT CCT TAC GGT ATC GAT GCT ACC GGT CAT ATC 460
GAC TAC GCC GAT CTG GAA AAA CAA GCC AAA GAA CAC AAG CCG 502
AAA ATG ATT ATC GGT GGT TTC TCT GCA TAT TCC GGC GTG GTG 544
GAC TGG GCG AAA ATG CGT GAA ATC GCT GAC AGC ATC GGT GCT 586
TAC CTG TTC GTT GAT ATG GCG CAC GTT GCG GGC CTG GTT GCT 628
GCT GGC GTC TAC CCG AAC CCG GTT CCT CAT GCT CAC GTT GTT 670
ACT ACC ACC ACT CAC AAA ACC CTG GCG GGT CCG CGC GGC GGC 712
CTG ATC CTG GCG AAA GGT GGT AGC GAA GAG CTG TAC AAA AAA 754
CTG AAC TCT GCC GTT TTC CCT GGT GGT CAG GGC GGT CCG TTG 796
ATG CAC GTA ATC GCC GGT AAA GCG GTT GCT CTG AAA GAA GCG 838
ATG GAG CCT GAG TTC AAA ACT TAC CAG CAG CAG GTC GCT AAA 880
AAC GCT AAA GCG ATG GTA GAA GTG TTC CTC GAG CGC GGC TAC 922
AAA GTG GTT TCC GGC GGC ACT GAT AAC CAC CTG TTC CTG GTT 964
GAT CTG GTT GAT AAA AAC CTG ACC GTT AAA GAA GCA GAC GCC1006
GCT CTG GGC CGT GCT AAC ATC ACC GTC AAC AAA AAC AGC GTA1048
CCG AAC GAT CCG AAG AGC CCG TTT GTG ACC TCC GGT ATT CGT1090
GTA GGT ACT CCG GCG ATT ACC CGT CGC GGC TTT AAA GAA GCC1132
GAA GCG AAA GAA CTG GCT GGC TGG ATG TGT GAC GTG CTG GAC1174
AGC ATC AAT GAT GAA GCC GTT ATC GAG CGC ATC AAA GGT AAA1216
GTT CTC GAC ATC TGC GCA CGT TAC CCG GTT TAC GCA 1252
TAAGCGAAAC GGTGATTTGC TGTCAATGTG CTCGTTGTTC ATGCCGGATG 1302
CGGCGTGAAC GCCTTATCCG GCCTACAAAA CTTTGCAAAT TCAATATATT 1352
GCAATCTCCG TGTAGGCCTG ATAAGCGTAG CGCATCAGGC AATTTTTCGT 1402
TTAT 1406

(2) INFORMATION FOR SEQ ID NO:2:
(i) SEQUENCE CHARACTERISTICS:
(A) LENGTH: 1408 nucleotides
(B) TYPE:nucleic acid
(C) STRANDEDNESS:single
(D) TOPOLOGY:linear
(ii) MOLECULE TYPE:DNA
(ix) FEATURES: reverse complement of SEQ ID NO:1 with
NdeI-BclI compatible ends
(xi) SEQUENCE DESCRIPTION:SEQ ID NO:2

GATCATAAAC GAAAAATTGC CTGATGCGCT ACGCTTATCA GGCCTACACG 50
GAGATTGCAA TATATTGAAT TTGCAAAGTT TTGTAGGCCG GATAAGGCAT 100
TCACGCCGCA TCCGGCATGA ACAACGAGCA CATTGACAGC AAATCACCGT 150
TTCGCTTATG CGTAAACCGG GTAACGTGCG CAGATGTCGA GAACTTTACC 200
TTTGATGCGC TCGATAACGG CTTCATCATT GATGCTGTCC AGCTCGTCAC 250
ACATCCAGCC AGCCAGTTCT TTCGCTTCGG CTTCTTTAAA GCCGCGACGG 300
GTAATCGCCG GAGTACCTAC ACGAATACCG GAGGTCACAA ACGGGCTCTT 350
CGGATCGTTC GGTGCGCTGT TTTTGTTGAC GGTGATGTTA GCACGGCCCA 400
GAGCGGCGTC TGCTTCTTTA ACAATCAGGT TTTTATCAAC CAGATCAACC 450
AGGAACAGGT GGTTATCAGT GCCGCCGGAA ACCACTTTGT AGCCGCGCTC 500
GAGGAACACT TCTACCATCG CTTTAGCGTT TTTAGCGACC TGCTGCTGGT 550
AAGTTTTGAA CTCAGGCTCC ATCGCTTCTT TCAGAGCAAC CGCTTTACCG 600
GCGATTACGT GCATCAACGG ACCGCCCTGA CCACCAGGGA AAACGGCAGA 650
GTTCAGTTTT TTGTACAGCT CTTCGCTACC ACCTTTCGCC AGGATCAGGC 700
CGCCGCGCGG ACCCGCCAGG GTTTTGTGAG TGGTGGTAGT AACAACGTGA 750
GCATGAGGAA CCGGGTTCGG GTAGACGCCA GCAGCAACCA GGCCCGCAAC 800
GTGCGCCATA TCAACGAACA GGTAAGCACC GATGCTGTCA GCGATTTCAC 850
GCATTTTCGC CCAGTCCACC ACGCCGGAAT ATGCAGAGAA ACCACCGATA 900
ATCATTTTCG GCTTGTGTTC TTTGGCTTGT TTTTCCAGAT CGGCGTAGTC 950
GATATGACCG GTAGCATCGA TACCGTAAGG AACGATGTTG TACAGTTTAC 1000

CGGAGAAGTT AACCGGAGAA CCGTGAGTCA GGTGTCCGCC ATGCGCCAGG 1050

TTCATACCCA GAACGGTATC ACCTGGTTCC AGCAGCGCGG TGTAGACCGC 1100

AAAGTTAGCC TGGGAGCCGG AGTGCGGCTG GACGTTAGCG TAGTCAGCGC 1150

CGAACAGTTC TTTCGCACGA TCGATCGCCA GTTGTTCAAC GATATCAACA 1200

TACTCGCAAC CGCCGTAGTA GCGTTTGCCC GGATAACCTT CAGCATATTT 1250

GTTGGTCAGC TGAGAACCCT GCGCCTGCAT TACGCGGGGG CTGGTGTAGT 1300

TTTCGGAGGC GATCAGTTCG ATGTGCTCTT CCTGACGTAC TTTTTCCTGC 1350

TCCATAGCCT GCCAGACTTC GGCATCATAA TCGGCAATGT TCATTTCACG 1400

CTTTAACA 1408

(2) INFORMATION FOR SEQ ID NO:3:
(i) SEQUENCE CHARACTERISTICS:
(A) LENGTH: 26 nucleotides
(B) TYPE:nucleic acid
(C) STRANDEDNESS:single
(D) TOPOLOGY:linear
(ii) MOLECULE TYPE:DNA
(xi) SEQUENCE DESCRIPTION:SEQ ID NO:3

GATGCATATGTTAAAGCGTGAAATGA 26

(2) INFORMATION FOR SEQ ID NO:4:
(i) SEQUENCE CHARACTERISTICS:
(A) LENGTH: 15 nucleotides
(B) TYPE:nucleic acid
(C) STRANDEDNESS:single
(D) TOPOLOGY:linear
(ii) MOLECULE TYPE:DNA
(xi) SEQUENCE DESCRIPTION:SEQ ID NO:4

CCGGAGAACCGTGAG 15

## Claims

1. A process for preparing an ultrahigh amount of *Escherichia coli* SHMT in soluble and active form within an *E. coli* host cell, said method comprising:
a) transforming the *E. coli* host cell with a recombinant DNA expression vector comprising:
i) a transcriptional activating sequence;
ii) a translational activating sequence; and
iii) a DNA sequence encoding *E. coli* SHMT;
such that i) ii) and iii) are operably linked to provide expression of *E. coli* SHMT; and
b) culturing the transformed *E. coli* host cell under conditions that allow for the expression of an ultrahigh amount of *E. coli* SHMT.

2. A process of Claim 1 wherein the transcriptional activating sequence is controlled by the bacteriophage lambda cI857 repressor.

3. A process of Claim 2 wherein the transcriptional activating sequence is selected from the group consisting of the wild-type bacteriophage lambda pL promoter and a modified bacteriophage lambda pL promoter.

4. A process of Claim 3 wherein the recombinant DNA expression vector is plasmid pZPl-glyA.

5. A process of Claim 1 wherein the host cell is selected from the group consisting of *Escherichia coli* K12 RV308/pZPl-glyA, and *Escherichia coli* K12 DH5α /pZPl-glyA.

6. A recombinant DNA expression vector capable of expressing an ultrahigh amount of *Escherichia coli* SHMT in a host cell transformed with the vector.

7. The recombinant DNA expression vector of Claim 6 that is plasmid pZPl-glyA.

8. A host cell transformed with a recombinant DNA expression vector of Claim 6.

9. A host cell transformed with the plasmid of Claim 8.

10. A transformed host cell of Claim 10 that is selected from the group consisting of *Escherichia coli* K12 RV308/pZPl-glyA, and *Escherichia coli* K12 DH5α /pZPl-glyA.
